# EUROPEAN PATENT APPLICATION

(11) **EP 4 467 644 A1**
(43) Date of publication of application: **27.11.2024**
(21) Application number: 22922190.8
(22) Date of filing: 22.12.2022
(51) Int. Cl.: C12N 15/11, C07F 9/6561

(54) **AMIDITE MONOMER**

(30) Priority: 21.01.2022 JP 2022007792
(71) Applicant: National University Corporation Tokai National Higher Education and Research System, Nagoya-shi, Aichi 464-8601 (JP)
(72) Inventor: ASANUMA, Hiroyuki, Nagoya-shi, Aichi 464-8601 (JP); KATO KAMIYA, Yukiko, Nagoya-shi, Aichi 464-8601 (JP); SATO, Fuminori, Nagoya-shi, Aichi 464-8601 (JP)
(74) Representative: Hoffmann Eitle
(86) International application number: PCT/JP2022/047371
(87) International publication number: WO 2023/140040

(57) **Abstract**

An object is to provide an amidite monomer of diaminopurine and/or an amidite monomer of a thiouracil derivative suitable for use in synthesis of an acyclic single-stranded polynucleotide comprising diaminopurine and/or a thiouracil derivative by a phosphoramidite method. This object is achieved by an amidite monomer of diaminopurine and/or an amidite monomer of a thiouracil derivative protected with a specific protecting group removable by a base.

## Description

### Technical Field

The present invention relates to an amidite monomer and the like.

### Background Art

Artificial polynucleotides, such as SNA (serinol nucleic acid) and aTNA (acyclic threoninol nucleic acid), have been reported to recognize DNA and RNA in a sequence-specific manner. Since these are acyclic polynucleotides that do not have a sugar skeleton, they are highly resistant to enzymatic degradation in vivo. They are also said to be easily synthesized. Therefore, acyclic polynucleotides are expected to be used as anti-miRNA, anti-mRNA, and siRNA, as well as molecular beacons.

### Citation List

### Patent Literature

PTL 1: WO2021/153762

### Summary of Invention

### Technical Problem

Acyclic polynucleotides comprising a palindromic structure represented by formula (2) : A¹-B-A² (wherein A¹ and A² are base sequences complementary to each other, and B is any base sequence) form self-duplexes even through there is some mismatch, which causes a problem in that they cannot bind to target polynucleotides (e.g., in vivo miRNA and mRNA). Patent Literature (PTL) 1 discloses that this problem can be solved by a single-stranded polynucleotide comprising a palindromic structure comprising an acyclic polynucleotide structural unit, wherein adenine in the palindromic structure is replaced by diaminopurine, and thymine at a position complementary to the adenine is replaced by a thiouracil derivative (e.g., 2-thiouracil or 2-thiothymine).

PTL 1 discloses that it was found that in synthesis of an acyclic single-stranded polynucleotide comprising diaminopurine and/or a thiouracil derivative by a phosphoramidite method, various impurities remain when conventional amidite monomers of diaminopurines and thiouracil derivatives are used, and that this problem can be solved by using protecting groups removable by acids as the protecting groups of bases in acyclic amidite monomers of diaminopurines and thiouracil derivatives.

However, in synthesis of an acyclic single-stranded polynucleotide comprising diaminopurine and/or a thiouracil derivative by a phosphoramidite method, the use of amidite monomers of diaminopurines and thiouracil derivatives protected with protecting groups removable by acids requires deprotection treatment to be performed in two steps, one for acids and one for bases, after synthesis. This causes problems in that purification using the hydrophobicity of the DMT group cannot be performed, and deprotection is insufficient in each operation, resulting in a decrease in oligomer yield.

An object of the present invention is to provide an amidite monomer of diaminopurine and/or an amidite monomer of a thiouracil derivative suitable for use in synthesis of an acyclic single-stranded polynucleotide comprising diaminopurine and/or a thiouracil derivative by a phosphoramidite method.

### Solution to Problem

The present inventors conducted extensive research to achieve the above object, and found that the use of an amidite monomer of diaminopurine and/or an amidite monomer of a thiouracil derivative protected with a specific protecting group removable by a base allows deprotection of the base moiety with higher efficiency and enables the desired acyclic polynucleotide to be obtained in a higher yield. As a result of further research based on this finding, the present inventors have completed the present invention. Specifically, the present invention includes the following embodiments.

### Item 1.

A compound or a salt thereof or a solvate thereof, the compound being represented by formula (1A) or (1B): 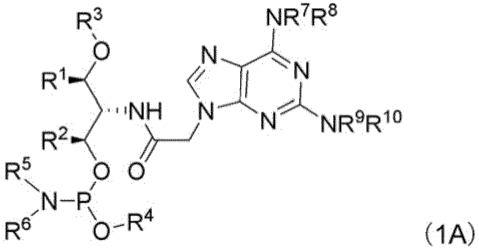 or 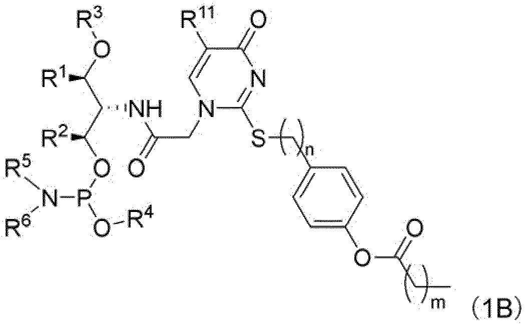 wherein
R¹ and R² are the same or different, and each is a hydrogen atom or an organic group;
R³ and R⁴ are the same or different, and each is a protecting group for a hydroxyl group;
R⁵ and R⁶ are the same or different, and each is an alkyl group;
R⁷, R⁸, R⁹, and R¹⁰ are the same or different, and each is a hydrogen atom or a protecting group removable by a base, excluding a case in which all are hydrogen atoms; and at least one protecting group removable by a base is a group represented by formula (a): 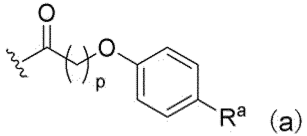
wherein R^{a} is a hydrogen atom or an alkyl group, and p is an integer of 1 to 3;
R¹¹ is a hydrogen atom or an alkyl group;
n is an integer of 1 to 3; and
m is an integer of 0 to 3.

### Item 2.

The compound or a salt thereof or a solvate thereof according to Item 1, wherein at least one of R⁷ and R⁸ is a group represented by formula (a), and at least one of R⁹ and R¹⁰ is a group represented by formula (a).

### Item 3.

The compound or a salt thereof or a solvate thereof according to Item 1 or 2, wherein n is 1, m is 0, R^{a} is a hydrogen atom, and p is 1.

### Item 4.

The compound or a salt thereof or a solvate thereof according to any one of Items 1 to 3, wherein R³ is a group represented by formula (b): 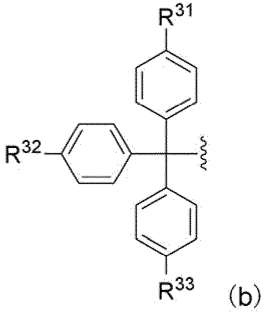 wherein R³¹, R³², and R³³ are the same or different, and each is a hydrogen atom or an alkoxy group, R⁴ is -(CH₂)₂-CN, and R⁵ and R⁶ are isopropyl groups.

### Item 5.

A reagent comprising the compound or a salt thereof or a solvate thereof according to any one of Items 1 to 4.

### Item 6.

The reagent according to Item 5, which is a reagent for producing a polynucleotide.

### Item 7.

A method for producing a single-stranded polynucleotide by a phosphoramidite method, the method comprising using the compound or a salt thereof or a solvate thereof according to any one of Items 1 to 4 as an amidite monomer.

### Advantageous Effects of Invention

The present invention provides an amidite monomer of diaminopurine and/or an amidite monomer of a thiouracil derivative suitable for use in synthesis of an acyclic single-stranded polynucleotide comprising diaminopurine and/or a thiouracil derivative by a phosphoramidite method. By synthesizing an acyclic single-stranded polynucleotide using the amidite monomer by a phosphoramidite method, deprotection of the base moiety can be performed with higher efficiency, and the desired acyclic polynucleotide can be obtained in a higher yield.

### Brief Description of Drawings

Fig. 1 is an MALDI-TOF-MS chart of the crude product of the SNA polynucleotide synthesized in Example 3.
Fig. 2 is an MALDI-TOF-MS chart of the crude product of the SNA polynucleotide synthesized in Example 4.

### Description of Embodiments

In the present specification, the terms "comprise" and "contain" include the concepts of comprising, containing, essentially consisting of, and consisting of.

### 1. Amidite Monomer

In an embodiment, the present invention relates to a compound represented by formula (1A) or (1B) or a salt thereof or a solvate thereof (which may be collectively referred to as "the compound of the present invention" in the present specification). This is described below.

Formula (1A) is the following formula.

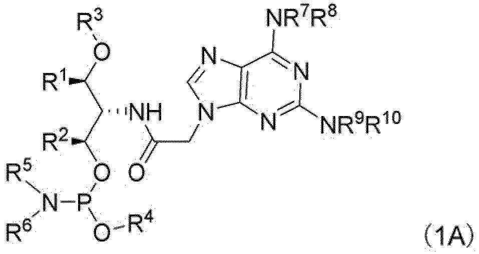

Formula (1B) is the following formula.

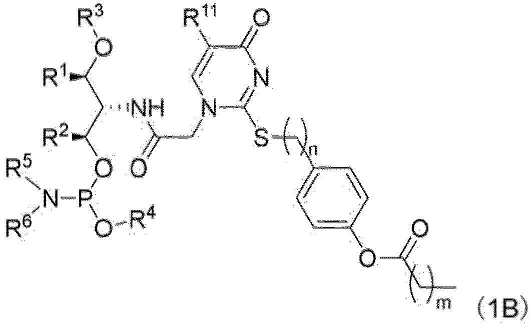

R¹ and R² are the same or different, and each is a hydrogen atom or an organic group.

The organic group represented by R¹ or R² is not particularly limited, and examples include hydrocarbon groups.

Preferred examples of the hydrocarbon group represented by R¹ or R² include chain hydrocarbon groups. Examples of chain hydrocarbon groups include alkyl, alkenyl, and alkynyl groups. Of these, alkyl groups are preferable. Specific examples of alkyl groups include methyl, ethyl, n-propyl, isopropyl, n-butyl, isobutyl, tert-butyl, sec-butyl, n-pentyl, neopentyl, n-hexyl, and 3-methylpentyl groups. The number of carbon atoms in the hydrocarbon group is not particularly limited. The number of carbon atoms is preferably 1 to 8, more preferably 1 to 6, even more preferably 1 to 4, still even more preferably 1 to 2, and particularly preferably 1. Further preferred is an alkyl group containing an alkynyl group (-C=C-, -C=CH) at the end or inside, which enables the introduction of various functional groups by click reactions etc.

In addition to the above examples, the organic group represented by R¹ or R² may be a monovalent group obtained by removing one hydrogen atom or functional group from various molecules, such as molecules used to modify polynucleotides. Examples of such molecules include polyethylene glycol chains, dye molecules, polycation (spermine), groove binders, amino groups, hydroxyl groups, thiol groups, metal ligands, photocleavable functional groups, sugar chains, and the like. These can be linked directly or indirectly to the skeleton of the above structural unit. For example, click reactions (e.g., the reaction of alkyne and azide described above) can be used for linkage.

Molecular modeling of acyclic polynucleotides, such as SNA and L-aTNA, suggests that using relatively large organic groups as R¹ and R² would not affect the duplex formation ability or their structures.

In a preferred embodiment of the present invention, it is preferred that at least one of R¹ and R² is a hydrogen atom.

In a preferred embodiment of the present invention, in terms of binding properties to target polynucleotides, such as miRNA, it is preferred that R¹ is a hydrogen atom or a chain hydrocarbon group and that R² is a hydrogen atom.

R³ and R⁴ are the same or different, and each is a protecting group for a hydroxyl group.

As R³, any group that can function as a protecting group for a hydroxyl group can be used without any restriction, and a wide range of known protecting groups used for amidite monomers can be used. R³ is preferably, for example, a group represented by formula (b).

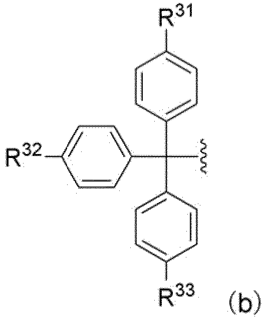

R³¹, R³², and R³³ are the same or different, and each is a hydrogen atom or an alkoxy group.

It is preferred that one of R³¹, R³², and R³³ is hydrogen, while the other two are alkoxy groups. Particularly preferred alkoxy groups are methoxy groups.

As R⁴, any group that can function as a protecting group for a hydroxyl group can be used without any restriction, and a wide range of known protecting groups used for amidite monomers can be used. Examples of R⁴ include alkyl, alkenyl, alkynyl, cycloalkyl, haloalkyl, aryl, heteroaryl, arylalkyl, cycloalkenyl, cycloalkylalkyl, cyclylalkyl, hydroxyalkyl, aminoalkyl, alkoxyalkyl, heterocyclylalkenyl, heterocyclylalkyl, heteroarylalkyl, silyl, silyloxyalkyl, mono-, di-, or trialkylsilyl, and mono-, di-, or trialkylsilyloxyalkyl groups. These may be substituted with electron-withdrawing groups.

R⁴ is preferably an alkyl group substituted with an electron-withdrawing group. Examples of the electron-withdrawing group include cyano, nitro, alkylsulfonyl, halogen, arylsulfonyl, trihalomethyl, and trialkylamino groups; a cyano group is preferred. R⁴ is particularly preferably -(CH₂)₂-CN.

R⁵ and R⁶ are the same or different, and each is an alkyl group.

The alkyl group represented by R⁵ or R⁶ may be linear or branched, and is preferably a C₁₋₁₂ alkyl group, and more preferably a C₁₋₆ alkyl group. Examples of alkyl groups include methyl, ethyl, n-propyl, isopropyl, n-butyl, isobutyl, tert-butyl, n-pentyl, isopentyl, and hexyl. The alkyl groups as mentioned herein include the alkyl moiety of an alkoxy group etc. R⁵ and R⁶ may be bonded together to form a ring structure.

R⁵ and R⁶ are particularly preferably both isopropyl groups.

R⁷, R⁸, R⁹, and R¹⁰ are the same or different, and each is a hydrogen atom or a protecting group removable by a base, excluding a case in which all are hydrogen atoms. At least one protecting group removable by a base is a group represented by formula (a).

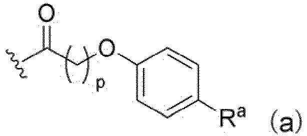

R^{a} is a hydrogen atom or an alkyl group. p is an integer of 1 to 3.

The alkyl group represented by R^{a} is linear or branched (preferably branched) and is preferably a C₁₋₁₂ alkyl group, and more preferably a C₁₋₆ alkyl group. Examples of alkyl groups include methyl, ethyl, n-propyl, isopropyl, n-butyl, isobutyl, tert-butyl, n-pentyl, isopentyl, and hexyl. The alkyl group represented by R^{a} is particularly preferably an isopropyl group.

R^{a} is particularly preferably a hydrogen atom.

p is particularly preferably 1.

In a preferred embodiment of the present invention, at least one of R⁷ and R⁸ is a group represented by formula (a), and at least one of R⁹ and R¹⁰ is a group represented by formula (a).

In addition to the group represented by formula (a), preferred examples of protecting groups removable by a base include, but are not particularly limited to, alkoxyacyl groups (e.g., methoxyacetyl), alkanoyl groups (e.g., isobutyryl and pivaloyl), a benzoyl group, and the like.

R¹¹ is a hydrogen atom or an alkyl group. n is an integer of 1 to 3. m is an integer of 0 to 3.

The alkyl group represented by R¹¹ may be linear or branched, and is preferably a C₁₋₁₂ alkyl group, and more preferably a C₁₋₆ alkyl group. Examples of alkyl groups include methyl, ethyl, n-propyl, isopropyl, n-butyl, isobutyl, tert-butyl, n-pentyl, isopentyl, and hexyl. The alkyl group represented by R^{a} is particularly preferably a methyl group.

R¹¹ is preferably a hydrogen atom or a methyl group, and more preferably a hydrogen atom.

n is particularly preferably 1.

m is particularly preferably 0.

In formula (1B), in place of the group represented by formula (1Ba): 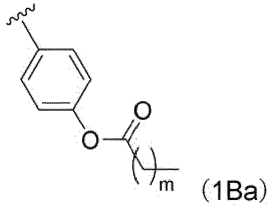 , a group represented by formula (1Bax): 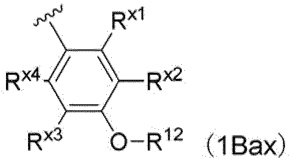 can also be used.

R¹² is a protecting group removable by a base. The protecting group removable by a base is not particularly limited, and may be, for example, a protecting group described above.

In an embodiment, R^{x1}, R^{x2}, R^{X3}, and R^{x4} are the same or different, and each may be a hydrogen atom or an organic group.

In another embodiment, R^{x1} and R^{x4} are the same or different, and each may be a hydrogen atom or electron-donating group; R^{x2} and R^{x3} are the same or different, and each may be a hydrogen atom or an electron-withdrawing group.

In place of the group represented by formula (a), a group represented by formula (ay): 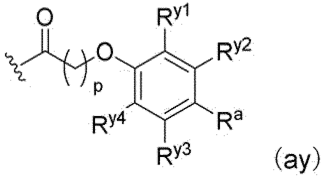 can also be used.

In an embodiment, R^{y1}, R^{y2}, R^{y3}, and R^{y4} are the same or different, and each may be a hydrogen atom or an organic group.

In another embodiment, R^{y1} and R^{y4} are the same or different, and each may be a hydrogen atom or an electron-donating group; R^{y2} and R^{y3} are the same or different, and each may be a hydrogen atom or an electron-withdrawing group.

The salt of the compound represented by formula (1A) or (1B) is not particularly limited, and examples include salts with inorganic bases, such as sodium salts, magnesium salts, potassium salts, calcium salts, and aluminum salts; salts with organic bases, such as methylamine, ethylamine, and ethanolamine; salts with basic amino acids, such as lysine, ornithine, and arginine; and ammonium salts. These salts may be acid addition salts. Specific examples of such salts include acid addition salts with mineral acids, such as hydrochloric acid, hydrobromic acid, hydriodic acid, sulfuric acid, nitric acid, and phosphoric acid; acid addition salts with organic acids, such as formic acid, acetic acid, propionic acid, oxalic acid, malonic acid, malic acid, tartaric acid, fumaric acid, succinic acid, lactic acid, maleic acid, citric acid, methanesulfonic acid, trifluoromethanesulfonic acid, and ethanesulfonic acid; and acid addition salts with acidic amino acids, such as aspartic acid and glutamic acid.

The solvate of the compound represented by formula (1A) or (1B) or a salt thereof is not particularly limited and is, for example, a solvate with a solvent, such as water, ethanol, glycerol, or acetic acid.

### 2. Method for Producing Amidite Monomer

The compound of the present invention can be produced by various methods.

An example of the compound represented by formula (1A) can be produced, for example, by using a protecting group-added base produced in accordance with or based on the following scheme, and then in accordance with or based on the method described in Example 1.

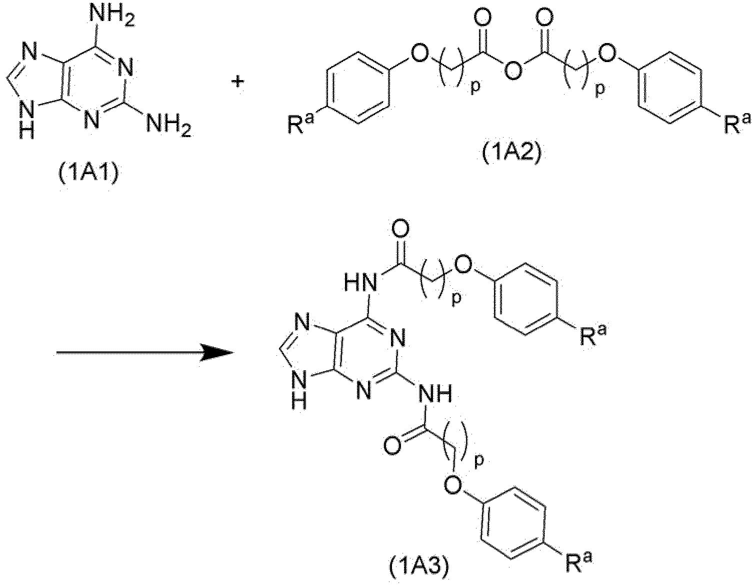

As the compound represented by formula (1A1) and the compound represented by formula (1A2), commercially available compounds can be directly used, or those synthesized in accordance with or based on known methods can be used as necessary.

In place of an anhydride, such as the compound represented by formula (1A2), an acid chloride (e.g., phenoxyacetyl chloride) can also be used.

The amount of the compound represented by formula (1A2) used is generally preferably 1 to 3 moles, and more preferably 1.5 to 2.5 moles, per mole of the compound represented by formula (1A1), in terms of yield, ease of synthesis, and the like.

This reaction is generally performed in the presence of a reaction solvent. Examples of reaction solvents include, but are not particularly limited to, pyridine, dimethylformamide, dichloromethane, acetonitrile, tetrahydrofuran, acetone, toluene, ethanol, and the like, with pyridine and the like being preferable. These solvents may be used singly or in a combination of two or more.

This reaction can also be performed in the presence of a base, such as N,N-dimethylaminopyridine.

In this reaction, in addition to the above components, additives can also be appropriately used as long as the progress of the reaction is not significantly impaired.

The reaction can be performed under heating, at room temperature, or under cooling, and is generally preferably performed at 10 to 100°C. The reaction time is not particularly limited and is generally 30 minutes to 30 hours.

The progress of the reaction can be traced by chromatography or other usual methods. After completion of the reaction, the solvent is distilled off, and the product can be isolated and purified by chromatography, recrystallization, or other usual methods. The structure of the product can be identified by elemental analysis, MS (ESI-MS) analysis, IR analysis, ¹H-NMR, ¹³C-NMR, etc.

An example of the compound represented by formula (1B) can be produced, for example, by using a protecting group-added base produced in accordance with or based on the following scheme (X is a halogen atom, such as -Cl, -Br, or -I; or an electron-withdrawing group, such as -CF₃, -CCl₃, -NO₂, -CN, or -OTs), and then in accordance with or based on the method described in Example 2.

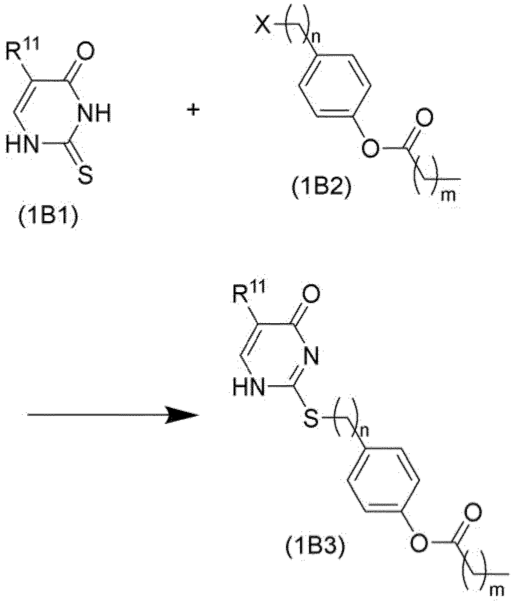

As the compound represented by formula (1B1), a commercially available compound can be directly used, or a compound synthesized in accordance with or based on a known method can be used as necessary.

As the compound represented by formula (1B2), a commercially available compound can be directly used, or a compound synthesized in accordance with or based on a known method and/or in accordance with or based on a method described in the Examples can be used as necessary.

The amount of the compound represented by formula (1B2) used is generally preferably 1 to 3 moles, and more preferably 1.5 to 2.5 moles, per mole of the compound represented by formula (1B1), in terms of yield, ease of synthesis, and the like.

This reaction is generally performed in the presence of a reaction solvent. Examples of reaction solvents include, but are not particularly limited to, ethanol, pyridine, dimethylformamide, dichloromethane, acetonitrile, tetrahydrofuran, acetone, toluene, and the like, with ethanol and the like being preferable. These solvents may be used singly or in a combination of two or more.

This reaction is preferably performed in the presence of a base, such as potassium hydroxide or triethylamine.

In this reaction, in addition to the above components, additives can also be appropriately used as long as the progress of the reaction is not significantly impaired.

The reaction can be performed under heating, at room temperature, or under cooling, and is generally preferably performed at 10 to 50°C. The reaction time is not particularly limited and is generally 30 minutes to 30 hours.

The progress of the reaction can be traced by chromatography or other usual methods. After completion of the reaction, the solvent is distilled off, and the product can be isolated and purified by chromatography, recrystallization, or other usual methods. The structure of the product can be identified by elemental analysis, MS (ESI-MS) analysis, IR analysis, ¹H-NMR, ¹³C-NMR, etc.

### 3. Reagent

In an embodiment, the present invention relates to a reagent comprising the compound of the present invention (which may be referred to as "the reagent of the present invention" in the present specification). This is described below.

The reagent of the present invention is not particularly limited as long as it comprises the compound of the present invention, and may comprise other components as necessary. The other components are not particularly limited as long as they are pharmaceutically acceptable components. The other components include not only components with pharmacological action, but also additives. Examples of additives include bases, carriers, solvents, dispersants, emulsifiers, buffers, stabilizers, excipients, binders, disintegrants, lubricants, thickeners, moisturizers, colorants, fragrances, chelating agents, and the like.

The content of the compound of the present invention in the reagent of the present invention is, for example, 0.001 to 100 mass%.

The usage of the reagent of the present invention is not particularly limited, and a suitable usage can be adopted depending on the type of reagent. The reagent of the present invention can be used, for example, as a reagent for producing a polynucleotide.

The reagent of the present invention may be in the form of a kit. The kit may comprise another reagent or an instrument (e.g., a reagent or instrument used in the production of polynucleotides), if necessary.

### 4. Method for Producing Single-stranded Polynucleotide

In an embodiment, the present invention relates to a method for producing a single-stranded polynucleotide by a phosphoramidite method, the method comprising using the compound of the present invention (which may be referred to as "the production method of the present invention" in the present specification). This is described below.

The phosphoramidite method can be performed by a known method using, for example, a commercially available nucleic acid automatic synthesizer. Specifically, the phosphoramidite method comprises, for example, (A) a step of deprotecting the hydroxyl group at the 5'-position (or an equivalent position) (in the case of the compound of the present invention, deprotection of R³), (B) a step of condensing the compound of the present invention, (C) a step of capping the hydroxyl group at the 5'-position (or an equivalent position) of the unreacted compound (in the case of the compound of the present invention, the hydroxyl group composed of R³ becoming a hydrogen atom by step (B)), (D) a step of converting the phosphite group to a phosphate group or a thiophosphate group, (E) a step of cleaving the obtained compound from the solid-phase carrier and deprotecting the phosphate moiety (in the case of the compound of the present invention, R⁴) and the nucleobase (in the case of the compound of the present invention, the protecting group(s) in R⁷ to R¹⁰), and (F) a step of deprotecting the hydroxyl group at the 5'-position (or an equivalent position) (in the case of the compound of the present invention, deprotection of R³). A compound comprising a polynucleotide skeleton having a desired chain length can be produced by repeating steps (A) to (D).

As the capping reagent used in step (C), it is preferable to use a compound corresponding to the structure of the group represented by formula (a) (the compound represented by formula 1A2 described above). This can further suppress deprotection deficiency and synthesis deficiency in step (E).

In step (D), it is preferable to use a solution containing iodine/water as an oxidizing agent.

In the deprotection in step (E), deprotection is performed with a base, such as ammonia water. In this case, it is preferable to add sodium hydrogensulfide (final concentration is preferably 20 to 100 mM, and more preferably 40 to 60 mM). This allows a side reaction (conversion reaction of thiocarbonyl of the nucleobase to amino) to be further suppressed. In the production method of the present invention, step (E) does not include a deprotection step with an acid.

After step (E), it is preferable to perform chromatography purification (e.g., reversed-phase chromatography purification) by utilizing the hydrophobicity of the protecting group (hydrophobic group) at the 5'-position before step (F). This allows the purity of the desired single-stranded polynucleotide to be further increased.

The obtained single-stranded polynucleotide can be isolated and purified, if necessary. In general, isolation can be achieved by precipitation, extraction, and purification of RNA. Specifically, RNA is precipitated by adding a solvent with low solubility for RNA, such as ethanol or isopropyl alcohol, to the solution after the reaction, or a solution of phenol, chloroform, and isoamyl alcohol is added to the reaction solution, and RNA is extracted into the aqueous layer. Then, isolation and purification can be achieved by known high-performance liquid chromatography (HPLC) techniques, such as reverse-phase column chromatography, anion-exchange column chromatography, and affinity column chromatography.

The single-stranded polynucleotide to be produced by the production method of the present invention is not particularly limited as long as it comprises a palindromic structure containing an acyclic polynucleotide structural unit and comprises diaminopurine and/or a thiouracil derivative (e.g., 2-thiouracil or 2-thiothymine) as a base.

The acyclic polynucleotide structural unit is a structural unit corresponding to the nucleotide constituting the polynucleotide and is not particularly limited as long as it does not contain a sugar skeleton. Typical examples of the acyclic polynucleotide structural unit include a structural unit represented by formula (1): 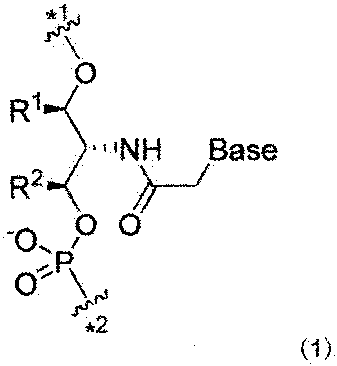 wherein R¹ and R² are as defined above, and Base is a nucleobase.

As the nucleobase, bases constituting nucleic acids can be used without restriction. Bases constituting nucleic acids include not only typical bases of natural nucleic acids, such as RNA and DNA (adenine (A), thymine (T), uracil (U), guanine (G), cytosine (C), etc.), but also other bases, such as hypoxanthine (I) and modified bases. Example of modified bases include pseudouracil, 3-methyluracil, dihydrouracil, 5-alkylcytosine (e.g., 5-methylcytosine), 5-alkyluracil (e.g., 5-ethyluracil), 5-halouracil (5-bromouracil), 6-azapyrimidine, 6-alkylpyrimidine (6-methyluracil), 4-acetylcytosine, 5-(carboxyhydroxymethyl)uracil, 5'-carboxymethylaminomethyl-2-thiouracil, 5-carboxymethylaminomethyluracil, 1-methyladenine, 1-methylhypoxanthine, 2,2-dimethylguanine, 3-methylcytosine, 2-methyladenine, 2-methylguanine, N6-methyladenine, 7-methylguanine, 5-methoxyaminomethyl-2-thiouracil, 5-methylaminomethyluracil, 5-methylcarbonylmethyluracil, 5-methyloxyuracil, 5-methyl-2-thiouracil, 2-methylthio-N6-isopentenyladenine, uracil-5-oxyacetic acid, 2-thiocytosine, purine, 2-aminopurine, isoguanine, indole, imidazole, xanthine, and the like.

In formula (1), *1 and *2 indicate the directions in which a polynucleotide is constituted. When R¹ is an organic group and R² is a hydrogen atom, *1 is the 3'-side and *2 is the 1'-side. When R¹ is a hydrogen atom and R² is an organic group, *1 is the 1'-side and *2 is the 3'-side. When R¹ is a hydrogen atom and R² is a hydrogen atom, *1 is the (S)-side and *2 is the (R)-side.

In the single-stranded polynucleotide, structural units other than the acyclic polynucleotide structural unit are not particularly limited, and structural units of natural nucleic acids and various artificial nucleic acids, including aTNA and SNA, can be used. In addition to DNA, RNA, etc., the nucleic acids that can be used may be specifically those that have undergone known chemical modifications, as shown below. In order to prevent degradation by hydrolytic enzymes, such as nucleases, phosphate residues (phosphates) of each nucleotide can be replaced by chemically modified phosphate residues, such as phosphorothioate (PS), methylphosphonate, and phosphorodithionate. Alternatively, the hydroxyl group at the 2'-position of the sugar (ribose) of each ribonucleotide may be replaced by -OR (R is, for example, CH₃ (2'-O-Me), CH₂CH₂OCH₃ (2'-O-MOE), CH₂CH₂NHC(NH)NH₂, CH₂CONHCH₃, CH₂CH₂CN, or the like) . Further, the base moiety (pyrimidine, purine) may be chemically modified; for example, a methyl group or a cationic functional group is introduced at the 5-position of the pyrimidine base, or the carbonyl group at the 2-position is replaced by thiocarbonyl. Other examples include, but are not limited to, modification of the phosphate moiety or hydroxyl moiety with, for example, biotin, an amino group, a lower alkyl amine group, an acetyl group, or the like. In addition, for example, BNA (LNA) in which the conformation of the sugar moiety is fixed to N-type by crosslinking 2'-oxygen and 4'-carbon in the sugar moiety of the nucleotide can also be preferably used.

It is patricianly preferable that the number of diaminopurines and the number of thiouracil derivatives are each 2 or more. The upper limit of this number is not particularly limited, and is, for example, 50, 20, 10, or 5.

The ratio of the sum of diaminopurines and thiouracil derivatives to 100% of the number of bases in the single-stranded polynucleotide is, for example, 10 to 90%, preferably 20 to 80%, more preferably 30 to 70%, and even more preferably 40 to 60%.

The ratio of the acyclic polynucleotide structural unit to 100% of the polynucleotide structural unit (the number of nucleotides) constituting the single-stranded polynucleotide is, for example, 70% or more, preferably 80% or more, more preferably 90% or more, even more preferably 95% or more, and particularly preferably 100% (i.e., the single-stranded polynucleotide of the present invention consists of the acyclic polynucleotide structural unit).

The base length of the single-stranded polynucleotide is not particularly limited, and is, for example, 6 to 10000. The base length is preferably 6 to 1000, more preferably 6 to 500, even more preferably 6 to 200, still even more preferably 6 to 100, and particularly preferably 6 to 50, in terms of ease of synthesis.

### Examples

The present invention is described in detail below based on Examples; however, the present invention is not limited to these Examples.

### Example 1: Synthesis of Pac₂D-SNA Amidite Monomer

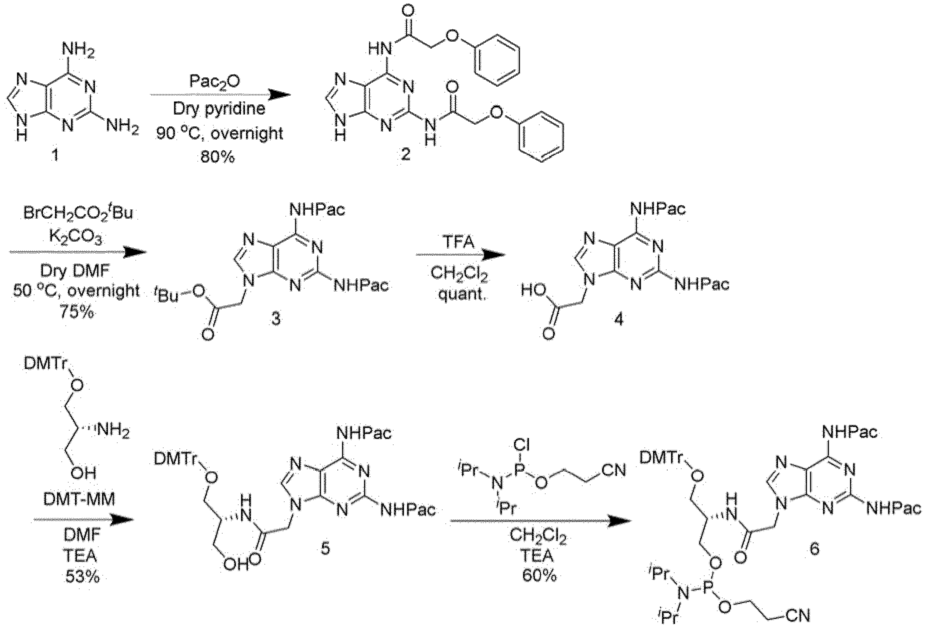

### Example 1-1: Synthesis of Compound 2

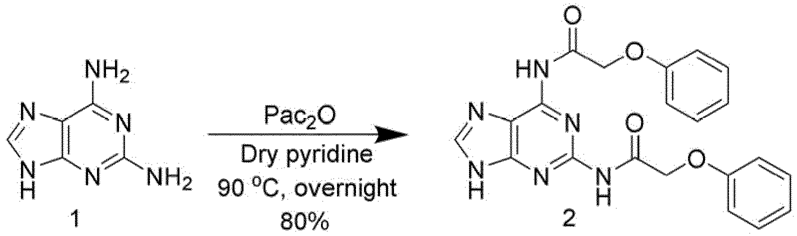

(1) 2,6-Diaminopurine (6.00 g, 40.0 mmol) and Pac₂O (25.0 g, 87.3 mmol, 2.2 eq) were placed in a 500 ml two-necked flask, and the flask was purged with nitrogen.
(2) 250 ml of dry pyridine was added, and the mixture was stirred on a hot-water bath at 90°C overnight (about 20 hours).
(3) The reaction solution was returned to room temperature and further cooled to 4°C.
(4) The reaction solution was suction-filtered. Washing was performed with cold pyridine, EtOH, and Et₂O, and the residue was vacuum-dried.
(5) Compound 2 was obtained as a white solid in an amount of 13.5 g (32.2 mmol) (yield: 80%).

### Example 1-2: Synthesis of Compound 3

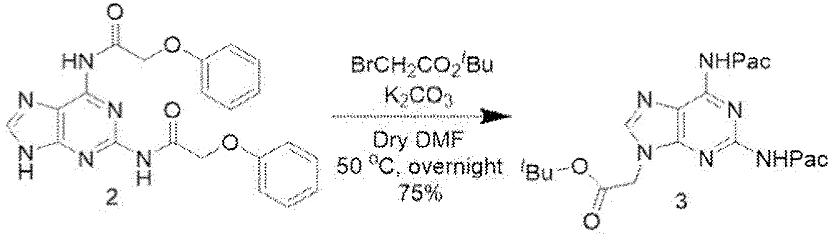

(1) Compound 2 (13.5 g, 32.2 mmol) and K₂CO₃ (4.90 g, 35.4 mmol, 1.10 eq) were placed in a two-necked flask, and the flask was purged with nitrogen.
(2) 150 ml of dry DMF was added, and the mixture was stirred well.
(3) tert-Butyl bromoacetate (5.20 ml, 35.4 mmol, 1.10 eq) was added at room temperature.
(4) The mixture was stirred on a hot-water bath at 50°C overnight (actually about 20 hours).
(5) DMF was dried up using an evaporator (PhMe azeotropic distillation).
(6) Suspension was performed in about 100 ml of ethanol, and the reaction solution was cooled and then suction-filtered (washed with cold EtOH).
(7) Washing was performed with H₂O, EtOH, and Et₂O in this order, and the residue was vacuum-dried.
(8) Compound 3 was obtained as a white solid in an amount of 12.8 g (24.0 mmol) (yield: 75%).

### Example 1-3: Synthesis of Compound 4

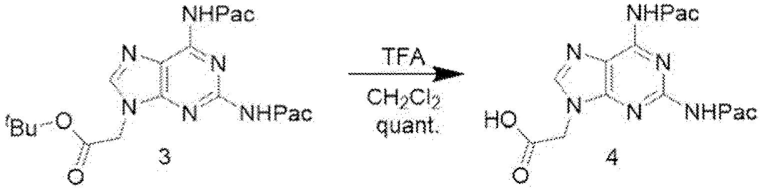

(1) Compound 3 (12.8 g, 24.0 mmol) was suspended in 90 ml of CH₂Cl₂.
(2) 180 ml of trifluoro acetic acid was added, and the mixture was vigorously stirred at room temperature for 2 hours.
(3) The reaction solution was concentrated under reduced pressure with an evaporator (azeotroped with CH₃CN)
(4) Suspension was performed well in about 200 ml of Et₂O, and suction filtration was performed.
(5) Washing was performed well with Et₂O, and the residue was vacuum-dried.
(6) Compound 4 was obtained as a white solid in an amount of 12.7 g (quant., 92 wt%).

### Example 1-4: Synthesis of Compound 5

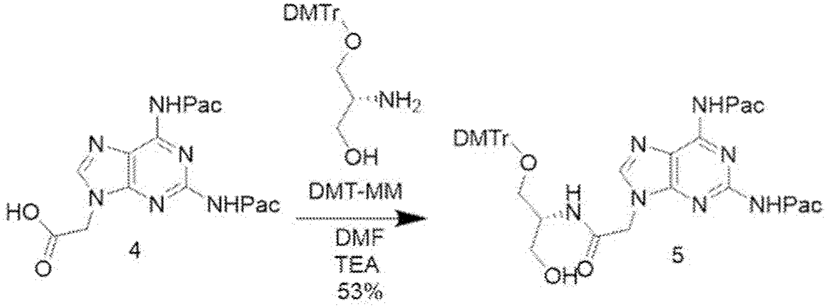

(1) Compound 4 (4.53 g, 8.0 mmol, 1.1 eq) was suspended in about 30 ml of DMF. Et₃N (4.43 ml, 40 mmol, 3.0 eq) was added, and the mixture was immersed in an ice bath.
(2) DMT-MM (11.2 mmol, 1.3 eq) was added, and the mixture was vigorously stirred in the ice bath for about 1 minute.
(3) SNA in DMF (8 mmol, 1.0 eq) was added dropwise, and the mixture was stirred in the ice bath for 30 minutes.
(4) The mixture was returned to room temperature and stirred for about another 30 minutes.
(5) After confirming the completion of the reaction by TLC (composition: CHCl₃:MeOH = 10:1; Rf value: about 0.5), about 100 ml of CHCl₃ was added, and the mixture was stirred for 10 minutes.
(6) Washing was performed with H₂O (150 ml x2), and brine (150 ml x1) .
(7) The organic layer was concentrated under reduced pressure (PhMe azeotropic distillation) and vacuum-dried.
(8) Purification was performed by silica gel column chromatography (CHCl₃:MeOH = 30:1 → CHCl₃:MeOH = 20:1, always 1% Et₃N).
(9) Azeotropic distillation was performed with CHCl₃:CH₃CN = 1:1 x1, CHCl₃ x1, and vacuum drying was performed.
(10) Compound 5 was obtained as a white solid in an amount of 3.59 g (4.21 mmol) (yield: 53%).

### Example 1-5: Synthesis of Compound 6

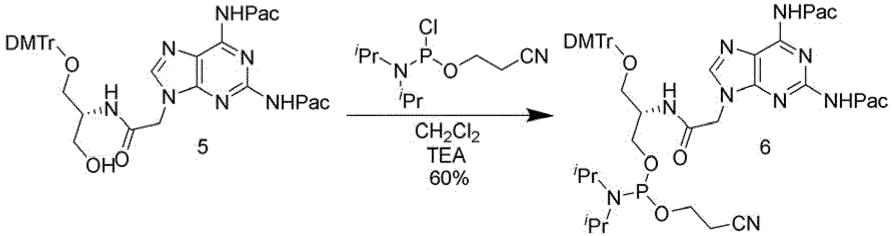

(1) Compound 5 (3.16 g, 3.70 mmol) was placed in a two-necked flask, and the flask was purged with nitrogen.
(2) Compound 5 was suspended well (not completely dissolved) in about 40 ml of dry CHCl₃. Et₃N (2.1 ml, 15 mmol, 4.0 eq) was added, and the mixture was stirred on an ice bath.
(3) An amiditing reagent (1.5 ml, greater than the amounts of other bases) was added dropwise on the ice bath, and the mixture was stirred at room temperature for about 30 minutes.
(4) After confirming the completion of the reaction, the liquid amount was reduced to about 10 ml to 20 ml by concentration under reduced pressure. The reaction solution was purified by silica gel column chromatography (CHCl₃:EtOAc = 2:1 → 1:1, containing 2% TEA) .
(5) The purified product was dissolved in a small amount of CHCl₃, and about 500 ml of n-hexane was added, followed by performing suspension well. Suction filtration was performed, and the residue was washed with n-hexane and vacuum-dried.
(6) Compound 6 was obtained as a white solid in an amount of 2.34 g (2.22 mmol) (yield: 60%).
¹H-NMR [DMSO, 500 MHz] δ = 10.93 (s, 1H), 10.61 (s, 1H), 8.41-8.39 (m, 1H), 8.26 (d, 1H), 7.38-7.35 (m,2H), 7.31-7.19 (m, 11H), 6.97-6.85 (m, 10H), 5.22 (s, 2H), 5.07 (s, 2H), 4.96-4.88 (m, 2H), 4.20-4.10 (m, 1H), 3.76-3.55 (m, 10H), 3.53-3.44 (m, 2H), 3.11-3.00 (m, 2H), 2.96-2.65 (m, 2H), 1.10 (dd, 6H), 1.03 (t, 6H). ¹³C{¹H} NMR [DMSO-d6, 125 MHz] δ 168.4, 165.9, 158.1, 157.97, 157.96, 152.9, 151.7, 148.8, 144.9, 144.6, 135.5, 129.72, 129.68, 129.42, 129.35, 127.8, 127.6, 126.7, 120.9, 120.7, 118.9, 118.1, 114.6, 114.4, 113.1, 85.38, 85.37, 67.8, 67.5, 61.9, 61.8, 61.7, 58.4, 58.33, 58.29, 58.2, 55.0, 50.2, 45.1, 42.5, 42.4, 24.3, 19.9, 19.8. ³¹P NMR [DMSO-d6, 202 MHz] δ 146.9, 146.6.. HRMS (FAB): Calcd for compound 6 (M+H⁺) 1052.4430; found 1052.4473.

### Example 2: Synthesis of 4-AcetoxybenzylsU-SNA Amidite Monomer

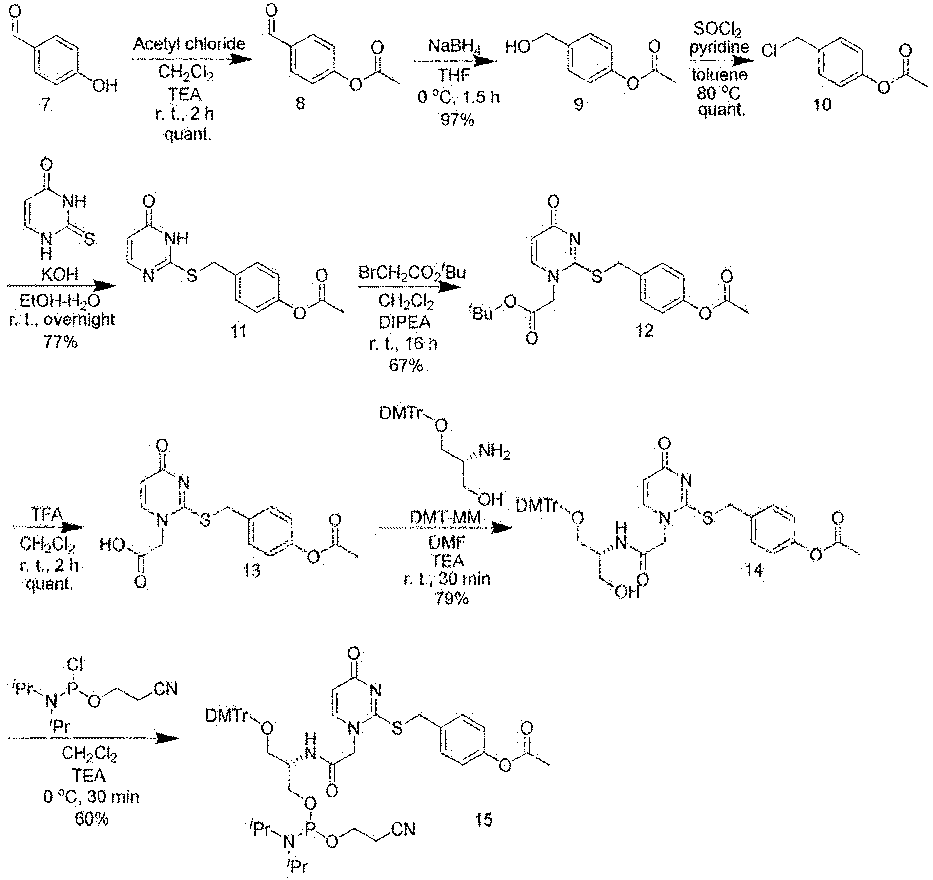

### Example 2-1: Synthesis of Compound 8

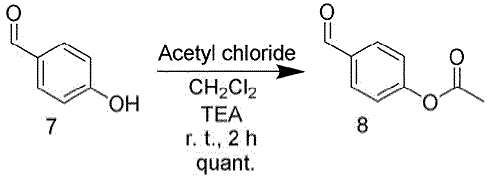

(1) 4-Hydroxybenzaldehyde (12.2 g, 100 mmol) was placed in a two-necked flask, and the flask was purged with nitrogen.
(2) About 200 ml of CH₂Cl₂ and Et₃N (41.9 ml, 300 mmol, 3.0 eq) were added, and the mixture was immersed in an ice bath and stirred.
(3) Acetyl chloride (10.7 ml, 150 mmol, 1.5 eq) was added dropwise.
(4) The mixture was stirred at room temperature for more than 2 hours.
(5) 50 ml of 1N HCl aq was added, and the mixture was stirred for about 20 minutes to quench the reaction.
(6) The organic layer was separated by liquid separation and washed with 1N HCl aq (50 ml x2).
(7) The organic layer was dried over magnesium sulfate and suction-filtered.
(8) The organic layer was concentrated under reduced pressure and vacuum-dried.
(9) Compound 8 was obtained as a brown viscous liquid in an amount of 21.6 g (quant.).

### Example 2-2: Synthesis of Compound 9

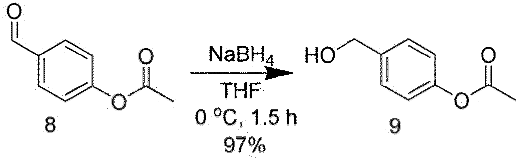

(1) Compound 8 (21.6 g) was placed in a 500 ml flask, dissolved in 150 ml of THF, and immersed in an ice bath.
(2) NaBH₄ (1.5 eq) was added, and the mixture was stirred for 1.5 hours.
(3) 150 ml of EtOAc was added thereto in the ice bath. Subsequently, 100 ml of satd. aq. NH₄Cl was slowly poured, and the mixture was stirred for 5 minutes.
(4) The organic layer was washed with satd. aq. NH₄Cl (100 ml x1).
(5) The organic layer was dried over magnesium sulfate and suction-filtered.
(6) The organic layer was concentrated under reduced pressure and vacuum-dried.
(7) Compound 9 was obtained as a yellow viscous liquid in an amount of 16.0 g (97 mmol) (yield: 97%).

### Example 2-3: Synthesis of Compound 10

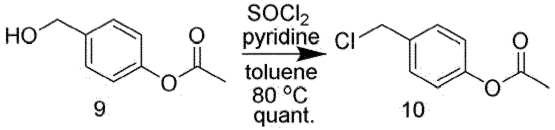

(1) Compound 9 (16.03 g, 96.5 mmol) was placed in a 500 ml two-necked flask, a reflux condenser was attached to the flask, and the flask was purged with nitrogen.
(2) 100 ml of dry toluene and 0.2 ml of dry pyridine were added, and the mixture was immersed in a hot-water bath at 80°C and stirred.
(3) Thionyl chloride (12 ml, 164 mmol, 1.7 eq) was added dropwise. After dropwise addition, the mixture was stirred for 1 hour.
(4) The reflux condenser was removed, the reaction solution was immersed in an ice bath, and 200 ml of satd. aq. NaHCO₃ was slowly poured together with finely crushed ice into the reaction solution while the reaction solution was stirred.
(5) When bubbling stopped, the organic layer was separated and washed with H₂O (100 ml x2) .
(6) The organic layer was dried over magnesium sulfate and suction-filtered.
(7) The organic layer was concentrated under reduced pressure and vacuum-dried.
(8) Compound 10 was obtained as a brown viscous liquid in an amount of 17.68 g (quant.).

### Example 2-4: Synthesis of Compound 11

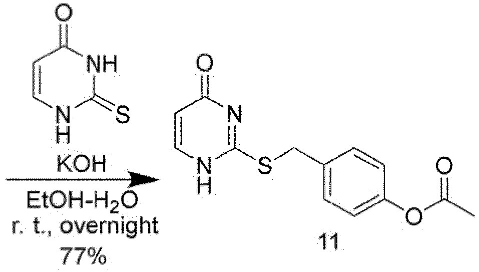

(1) 2-Thiouracil (6.34 g, 49.4 mmol) was suspended in 60 ml of EtOH.
(2) A >85% KOH (3.6 g, 54.7 mmol, in H₂O 60 ml) solution was added to (1) and heated to 45°C, followed by sonication, thereby completely dissolving 2-thiouracil.
(3) The resulting product of (2) was returned to room temperature and stirred. Compound 10 (96.5 mmol, 1.95 eq) was added dropwise using a Pasteur pipette. The remaining compound 10 was washed with about 6 ml of EtOH and added to the reaction solution, followed by stirring at room temperature overnight.
(4) 50 ml of NaHCO₃ aq and 100 ml of H₂O were added sequentially while the reaction solution was stirred.
(5) Suction filtration was performed, and the residue was washed sequentially with cold H₂O, cold EtOH, EtOAc, and Et₂O.
(6) Compound 11 was obtained as a white solid in an amount of 10.52 g (38.1 mmol) (yield: 77%).

### Example 2-5: Synthesis of Compound 12

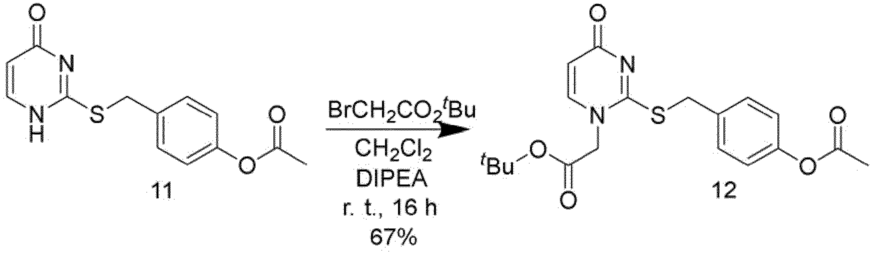

(1) Compound 11 (10.5 g, 38.1 mmol) was placed in a two-necked flask, and the flask was purged with nitrogen.
(2) 20 ml of dry CH₂Cl₂ and DIPEA (7.12 ml, 41.9 mmol, 1.1 eq) were added. The obtained suspension was immersed in an ice bath, followed by stirring.
(3) tert-Butyl bromoacetate (6.14 ml, 41.9 mmol, 1.1 eq) was added dropwise, and the mixture was stirred in the ice bath for 10 minutes.
(4) The mixture was stirred at room temperature for 16 hours.
(5) After confirming the completion of the reaction by TLC (composition: CHCl₃:MeOH = 10:1, Rf value: 0.4 to 0.5), 140 ml of H₂O was added to the reaction solution, and the mixture was stirred for 10 minutes.
(6) The organic layer was separated and washed with H₂O and brine. The organic layer was dried over magnesium sulfate and suction-filtered.
(7) The organic layer was concentrated under reduced pressure and vacuum-dried.
(8) The crude product was purified by silica gel column chromatography (composition: CHCl₃:MeOH = 30:1 → 20:1).
(9) Compound 12 was obtained as a white solid in an amount of 10.0 g (25.6 mmol) (yield: 67%).

### Example 2-6: Synthesis of Compound 13

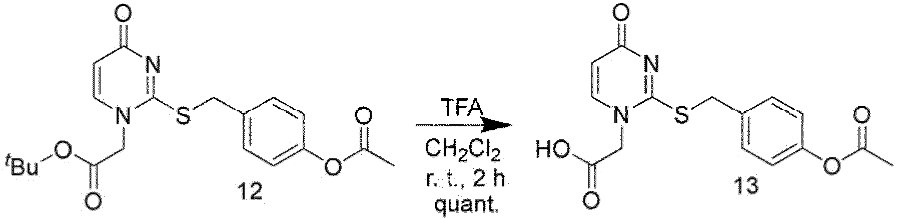

(1) Compound 11 (10.0 g, 25.6 mmol) was placed in a flask and dissolved in 60 ml of CH₂Cl₂ and 180 ml of TFA, followed by stirring at room temperature for 2 hours.
(2) Concentration under reduced pressure was performed, followed by suspension in Et₂O. Suction filtration was performed, and vacuum drying was performed.
(3) Compound 13 was obtained as a white solid in an amount of 10.0 g (quant.).

### Example 2-7: Synthesis of Compound 14

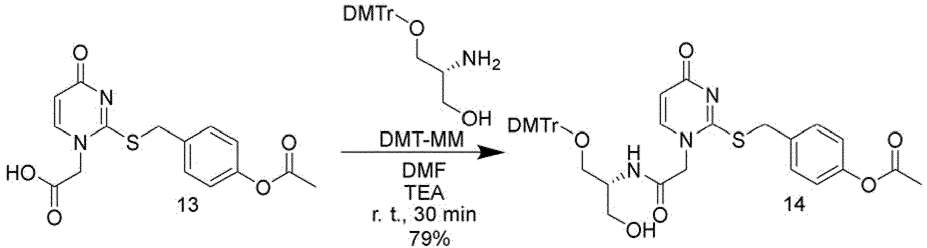

(1) Compound 13 (2.94 g, 8.8 mmol, 1.1 eq) was suspended in about 10 ml of DMF. Et₃N (5.54 ml, 40 mmol, 5.0 eq) was added, and the mixture was immersed in an ice bath.
(2) DMT-MM (11.2 mmol, 1.4 eq) was added, and the mixture was vigorously stirred in the ice bath for about 1 minute.
(3) SNA in DMF (8 mmol, 1.0 eq) was added dropwise, and the mixture was stirred in the ice bath for 10 minutes.
(4) The mixture was returned to room temperature and stirred for about another 30 minutes.
(5) After confirming the completion of the reaction by TLC (composition: CHCl₃:MeOH = 5:1; Rf value: about 0.5), the reaction solution was concentrated under reduced pressure.
(6) Dissolution and/or suspension in about 100 ml of CHCl₃ was performed, followed by stirring for 10 minutes.
(7) Washing was performed with NaHCO₃ aq (150 ml x2) and brine (150 ml x1).
(8) The organic layer was concentrated under reduced pressure and vacuum-dried.
(9) Purification was performed by silica gel column chromatography (CHCl₃:MeOH = 20:1 -. CHCl₃:MeOH = 10:1, always 2% Et₃N).
(10) Azeotropic distillation was performed with CH₃CN = 1:1 x1 and CHCl₃ x1, and vacuum drying was performed.
(11) Compound 5 was obtained as a white solid in an amount of 4.6 g (6.35 mmol) (yield: 79%).

### Example 2-8: Synthesis of Compound 15

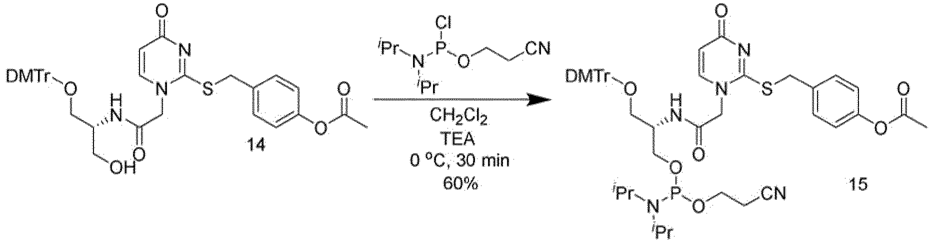

(1) Compound 5 (4.6 g, 6.35 mmol) was placed in a two-necked flask, and the flask was purged with nitrogen.
(2) Compound 5 was suspended well in about 8 ml of dry CH₂Cl₂, Et₃N (2.1 ml, 15 mmol, 5.0 eq) was added, and the mixture was stirred on an ice bath.
(3) An amiditing reagent (2.13 ml) was added dropwise on the ice bath. The mixture was stirred for about 30 minutes.
(4) After confirming the completion of the reaction, the liquid amount was reduced to about 10 ml to 20 ml by concentration under reduced pressure. The reaction solution was purified by silica gel column chromatography (CHCl₃:(CH₃)₂CO = 6:1 → 4:1 → 2:1, containing 2% TEA).
(5) The purified product was dissolved in a small amount of CHCl₃, and about 500 ml of n-hexane was added, followed by performing suspension well. Suction filtration was performed, and the residue was washed with n-hexane and vacuum-dried.
(6) Compound 15 was obtained as a white solid in an amount of 3.5 g (3.79 mmol) (yield: 60%).
¹H-NMR [CDCl₃, 500 MHz] δ= 7.40 (d, 2H), 7.31-7.25 (m, 8H), 7.22-7.18 (m, 1H), 7.13 (dd, 1H), 6.98-6.95 (m, 2H), 6.84-6.80 (m, 4H), 6.44 (t, 1H), 6.05 (dd, 1H), 4.45-4.29 (m, 5H), 3.92-3.45 (m, 12H), 3.36-3.28 (m, 1H), 3.19-3.15 (m, 1H), 2.54-2.36 (m, 2H), 2.81 (d, 3H), 1.17-1.06 (m, 12H). ¹³C{¹H} NMR [CDCl₃, 125 MHz] δ 169.54, 169.51, 167.9, 164.4, 164.3, 162.81, 162.77, 158.7, 150.3, 144.8, 144.7, 144.3, 144.2, 135.94, 135.86, 135.8, 133.0, 133.0, 130.7, 130.21, 130.17, 128.2, 128.02, 128.00, 127.0, 123.0, 122.0, 118.9, 113.3, 110.1, 86.3, 62.6, 62.5, 61.8, 61.7, 58.6, 58.5, 58.43, 58.36, 55.38, 55.37, 54.3, 54.2, 50.63, 50.57, 50.5, 43.2, 43.1, 35.9, 24.8, 24.7, 21.3 20.80, 20.76, 20.73, 20.69. ³¹P NMR [CDCl₃, 202 MHz] δ 147.9, 147.6. HRMS (FAB): Calcd for compound 6 (M+H⁺) 910.3609; found 910.3628.

### Example 3: Synthesis 1 of Single-stranded Polynucleotide

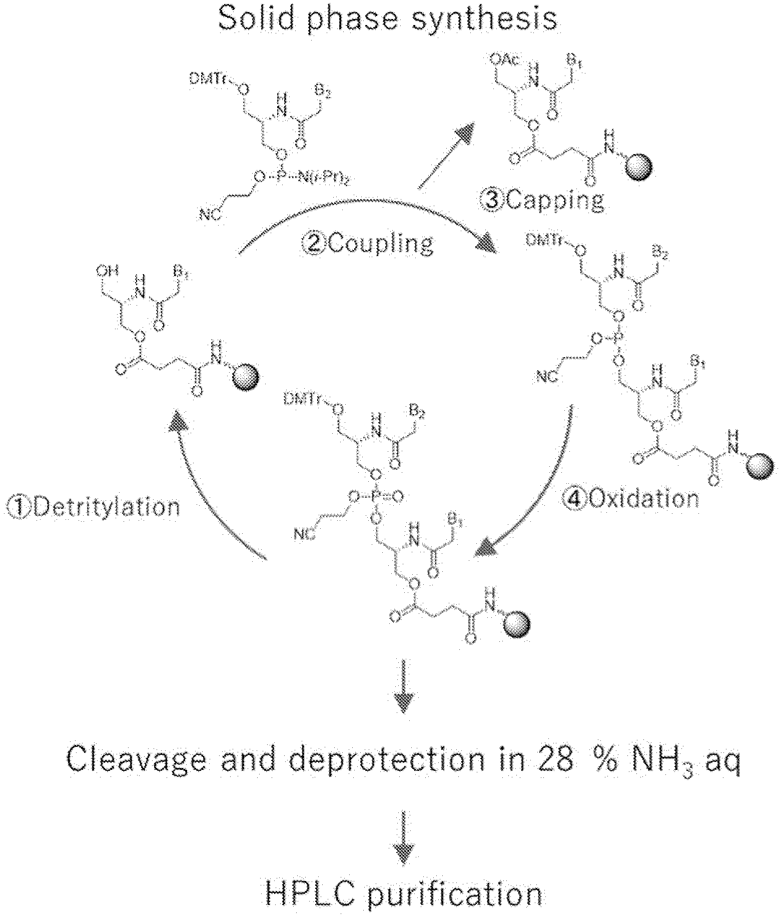

Based on the above scheme, an SNA polynucleotide containing 2,6-diaminopurine (D) as a base (base sequence: (S)-TTTDTTDT-(R)) was synthesized with a solid-phase synthesizer using the amidite monomer (Pac₂D-SNA amidite monomer, compound 6) synthesized in Example 1 by a phosphoramidite method. Phenoxyacetic anhydride, rather than acetic anhydride, was used as a capping reagent. After synthesis, cleavage and deprotection were performed in NH₃ aq at 55°C for 2 hours.

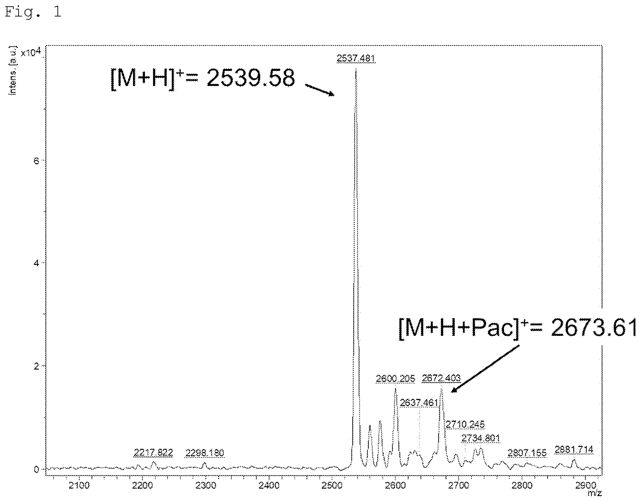

Fig. 1 is an MALDI-TOF-MS chart of the crude product (before HPLC purification in the above scheme). The peak of the sequence of interest ([M+H]⁺) (including ion coordination) was observed at high intensity, and only slight deprotection insufficiency was observed for the other peaks. Regarding the deprotection insufficiency, it was speculated that deprotection would be achieved without any problem if the deprotection time were extended.

### Example 4: Synthesis 2 of Single-stranded Polynucleotide

Based on the scheme of Example 3, an SNA polynucleotide containing 2,6-diaminopurine (D) and 2-thiouracil (sU) as bases (base sequence: (S)-CDD CDsU CDG TCsU G DsU DDG CTA-(R) (SEQ ID NO: 1)) was synthesized with a solid-phase synthesizer using the amidite monomer (Pac₂D-SNA amidite monomer, compound 6) synthesized in Example 1 and the amidite monomer (4-acetoxybenzylsU-SNA amidite monomer, compound 15) synthesized in Example 2 by a phosphoramidite method. Phenoxyacetic anhydride, rather than acetic anhydride, was used as a capping reagent. After synthesis, cleavage and deprotection were performed in NH₃ aq (containing 50 mM sodium hydrogensulfide) at 55°C for 3 hours.

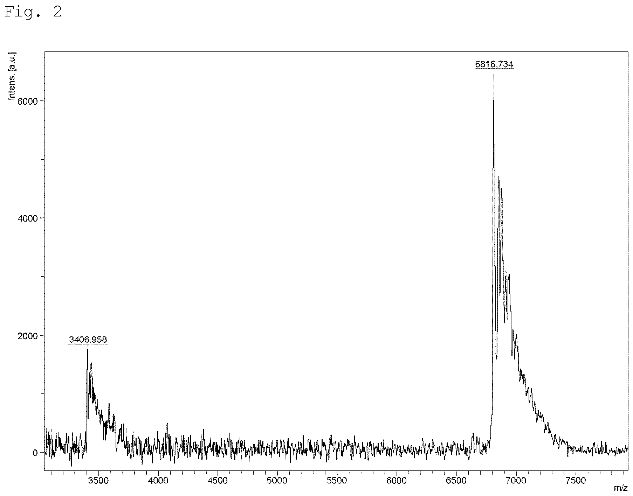

Fig. 2 is an MALDI-TOF-MS chart of the crude product (before HPLC purification in the scheme of Example 3). The peak of the sequence of interest ([M+H]⁺) (including ion coordination) was observed at high intensity.

### Comparative Example 1: Investigation 1 of Other Amidite Monomers

Amidite monomers having a structure that is the same as that of the amidite monomer (compound 6) synthesized in Example 1 except that the base moiety was any of the following groups were synthesized.

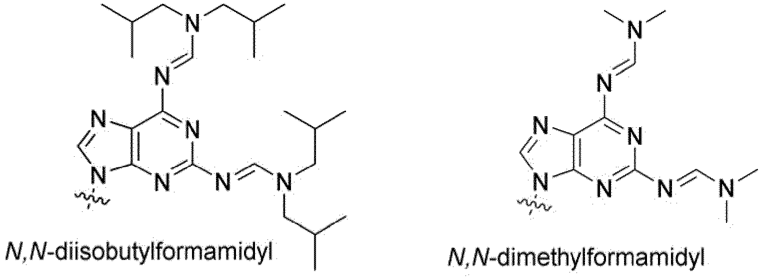

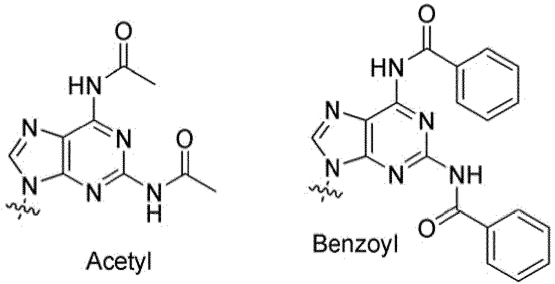

SNA polynucleotides containing 2,6-diaminopurine (D) as a base were synthesized with a solid-phase synthesizer using the amidite monomers by a phosphoramidite method as in Example 3. After synthesis, cleavage and deprotection were performed in NH₃ aq at 55°C. In this case, the reaction was carried out for a long period of time (about 10 hours). In MS analysis of the crude products, peaks indicating that the deprotection was insufficient or peaks that were presumably reaction intermediates were strongly observed. Performing the reaction for a longer period of time leads to progress of hydrolysis of the SNA skeleton, resulting in a decrease in yield.

Synthesis of an amidite monomer having a structure that is the same as that of the amidite monomer (compound 6) synthesized in Example 1 except that the base moiety was the following group was attempted. However, during the process of synthesis of the amidite monomer, the protecting groups for the amino groups in the following group were deprotected, and the amidite monomer could not be synthesized.

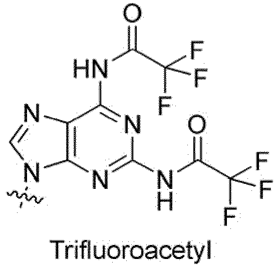

### Comparative Example 2: Investigation 2 of Other Amidite Monomers

Based on the following reaction scheme, the addition of various protecting groups to the thiocarbonyl of thiouracil was investigated.

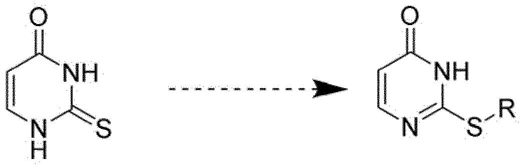

### Investigation 1: Cyanoethyl Group

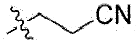

### Reaction Conditions

1: 3-Bromopropionitrile, Et₃N, DMF
2: 3-Bromopropionitrile, NaH, DMF
3: 3-Bromopropionitrile, K₂CO₃ DMF
4: 3-Bromopropionitrile, KOH, H₂O/EtOH (1:1)

Under any of the conditions, it was not possible to add a cyanoethyl group to the 2-thiocarbonyl group because of no reaction, or elimination after reaction (because it was a protecting group that is very easily removed).

### Investigation 2: Acetyl Group

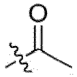

1: Acetyl chloride, Et₃N, DMF
2: Acetyl chloride, NaH, DMF
3: Acetyl chloride, KOH, H₂O/EtOH (1:1)

Under any of the conditions, it was not possible to obtain a compound in which an acetyl group is added to the 2-thiocarbonyl group because of no reaction, or elimination due to the following side reaction after reaction.

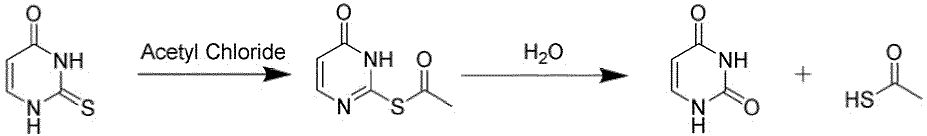

### Investigation 3: Triisopropylsilyl (TIPS) Group

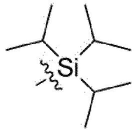

1: TIPS-Cl, 1H-imidazole, DMF
2: TIPS-Cl, NaH, DMF
3: TIPS-Cl, KOH, H₂O/EtOH (1:1)

Under any of the conditions, it was not possible to obtain a compound in which a TIPS group is added to the 2-thiocarbonyl, because of no reaction, or elimination after reaction.

### Example 5: Synthesis of Pac₂D-L-aTNA Amidite Monomer

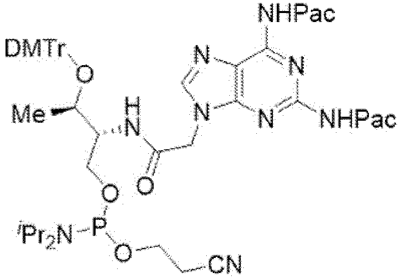

The above compound (Pac₂D-L-aTNA amidite monomer) was synthesized in the same manner as in compound 6 (Pac₂D-SNA amidite monomer) of Example 1. Specifically, the compound was synthesized in the same manner as in Example 1, except that L-aTNA was used in place of SNA in (2) of Example 1-4.

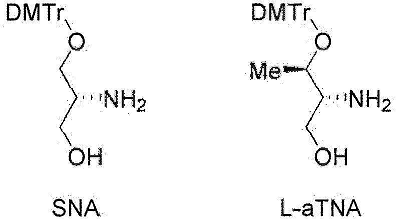

The Pac₂D-L-aTNA amidite monomer was obtained as a white solid in an amount of 1.65 g (1.56 mmol) (yield: 57%). ¹H NMR [DMSO-d6, 500 MHz] δ 10.93 (s, 1H), 10.63 (s, 1H), 8.32 (dd, 2H), 7.46 (dd, 2H), 7.36-7.24 (m, 10H), 7.12 (t, 1H), 6.98-6.85 (m, 10H), 5.23 (s, 2H), 5.12 (s, 2H), 5.02 (dd, 2H), 3.97-3.88 (m, 1H), 3.74-3.59 (m, 10H), 3.55-3.42 (m, 3H), 2.67 (dt, 2H), 1.12 (dd, 6H), 1.04 (dd, 6H), 0.70 (dd, 3H). ¹³C{¹H} NMR [DMSO-d6, 125 MHz] δ 168.6, 166.7, 166.6, 158.5, 158.5, 158.4, 153.4, 152.1, 149.2, 147.0, 146.8, 145.2, 137.0, 136.9, 136.9, 130.54, 130.48, 129.9, 129.8, 128.3, 128.0, 127.02, 126.97, 121.3, 121.2, 119.4, 118.6, 115.0, 114.9, 113.4, 86.1, 86.0, 69.3, 68.9, 68.3, 68.0, 59.0, 58.9, 58.7, 55.5, 42.93, 42.90, 42.84, 42.80, 24.83, 24.78, 24.7, 20.3, 20.3, 18.1, 17.9. ³¹P NMR [DMSO-d6, 202 MHz] δ 147.0, 146.9. HRMS (FAB): Calcd for C₅₇H₆₅N₉O₁₀P (Pac₂D-L-aTNA amidite monomer) [M + H]⁺, 1066.4587; found, 1066.4605.

### Example 6: Synthesis of 4-AcetoxybenzylsU-L-aTNA Amidite Monomer

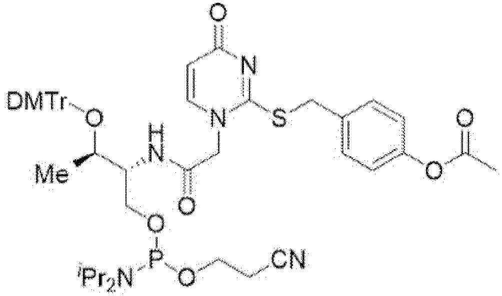

The above compound (4-acetoxybenzylsU-L-aTNA amidite monomer) was synthesized in the same manner as in compound 15 (4-acetoxybenzylsU-SNA amidite monomer) of Example 2. Specifically, the compound was synthesized in the same manner as in Example 2, except that L-aTNA was used in place of SNA in (2) of Example 2-7.

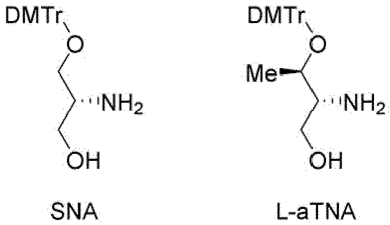

The 4-acetoxybenzylsU-L-aTNA amidite monomer was obtained as a white solid in an amount of 7.01 g (7.59 mmol) (yield: 83%).
¹H NMR [CDCl₃, 500 MHz] δ 7.44 (d, 2H), 7.36-7.30 (m, 6H), 7.22 (m, 2H), 7.18-7.12 (m, 2H), 6.97 (d, 2H), 6.82-6.73 (m, 5H), 6.04 (dd, 1H), 4.50-4.37 (m, 4H), 4.02-3.95 (m, 1H), 3.80-3.57 (m, 11H), 3.54-3.44 (m, 3H), 2.52-2.45 (m, 2H), 2.26 (s, 3H), 1.13 (dd, 6H), 1.06 (dd, 6H), 0.91 (dd, 3H). ¹³C{¹H} NMR [CDCl₃, 125 MHz] δ 169.4, 167.9, 164.6, 164.5, 162.7, 158.52, 158.49, 150.2, 146.3, 146.2, 144.54, 144.50, 136.8, 136.5, 132.8, 130.5, 130.42, 130.40, 130.3, 128.13, 128.11, 127.7, 126.8, 121.9, 121.8, 118.5, 113.04, 113.01, 109.9, 109.8, 86.3, 69.0, 68.9, 62.8, 62.63, 62.56, 62.45, 58.4, 58.34, 58.26, 58.2, 55.50, 55.45, 55.2, 54.3, 54.2, 43.0, 42.9, 35.8, 24.61, 24.59, 24.55, 21.1, 20.52, 20.48, 20.45, 18.7, 18.6. ³¹P NMR [CDCl₃, 202 MHz] δ 147.9, 147.3. HRMS (FAB) : Calcd for C₄₉H₅₉N₅O₉PS (4-AcetoxybenzylsU-L-aTNA amidite monomer) [M + H]⁺, 924.3766; found, 924.3797.

### Example 7: Synthesis 3 of Single-stranded Polynucleotide

Based on the scheme of Example 3, an L-aTNA polynucleotide containing 2,6-diaminopurine (D) and 2-thiouracil (sU) as bases was synthesized with a solid-phase synthesizer using the amidite monomer (Pac₂D-L-aTNA amidite monomer) synthesized in Example 5 and the amidite monomer (4-acetoxybenzylsU-L-aTNA amidite monomer) synthesized in Example 6 by a phosphoramidite method. Phenoxyacetic anhydride, rather than acetic anhydride, was used as a capping reagent. After synthesis, cleavage and deprotection were performed in NH₃ aq (containing 50 mM sodium hydrogensulfide) at 55°C for 3 hours.

The MALDI-TOF-MS chart was checked as in the case of the SNA polynucleotide of Example 4, and the peak of the sequence of interest ([M+H]⁺) (including ion coordination) was observed at high intensity.

## Claims

1. A compound or a salt thereof or a solvate thereof, the compound being represented by formula (1A) or (1B): 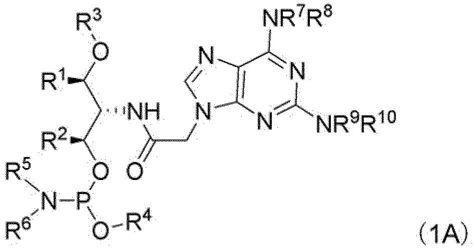 or 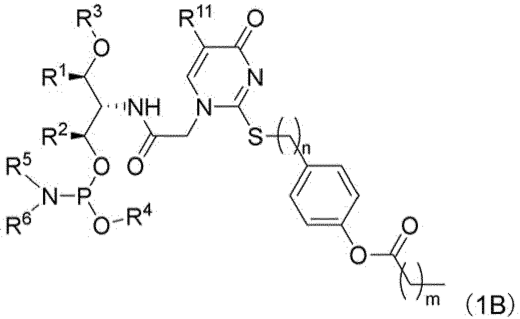 wherein
R¹ and R² are the same or different, and each is a hydrogen atom or an organic group;
R³ and R⁴ are the same or different, and each is a protecting group for a hydroxyl group;
R⁵ and R⁶ are the same or different, and each is an alkyl group;
R⁷, R⁸, R⁹, and R¹⁰ are the same or different, and each is a hydrogen atom or a protecting group removable by a base, excluding a case in which all are hydrogen atoms; and at least one protecting group removable by a base is a group represented by formula (a): 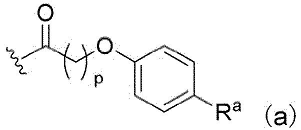
wherein R^{a} is a hydrogen atom or an alkyl group, and p is an integer of 1 to 3;
R¹¹ is a hydrogen atom or an alkyl group;
n is an integer of 1 to 3; and
m is an integer of 0 to 3.

2. The compound or a salt thereof or a solvate thereof according to claim 1, wherein at least one of R⁷ and R⁸ is a group represented by formula (a), and at least one of R⁹ and R¹⁰ is a group represented by formula (a).

3. The compound or a salt thereof or a solvate thereof according to claim 1 or 2, wherein n is 1, m is 0, R^{a} is a hydrogen atom, and p is 1.

4. The compound or a salt thereof or a solvate thereof according to any one of claims 1 to 3, wherein R³ is a group represented by formula (b): 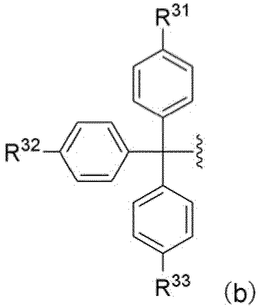 wherein R³¹, R³², and R³³ are the same or different, and each is a hydrogen atom or an alkoxy group, R⁴ is -(CH₂)₂-CN, and R⁵ and R⁶ are isopropyl groups.

5. A reagent comprising the compound or a salt thereof or a solvate thereof according to any one of claims 1 to 4.

6. The reagent according to claim 5, which is a reagent for producing a polynucleotide.

7. A method for producing a single-stranded polynucleotide by a phosphoramidite method, the method comprising using the compound or a salt thereof or a solvate thereof according to any one of claims 1 to 4 as an amidite monomer.
